# FASCICULE DE BREVET EUROPEEN

(11) **EP 0 720 848 B1**
(45) Date de publication et mention de la délivrance du brevet: **12.03.2003**
(21) Numéro de dépôt: 95440090.9
(22) Date de dépôt: 21.12.1995
(51) Int. Cl.: A61K 7/48, A61K 35/78

(54) **Utilisation d'extraits de graines de tamarin enrichis en xyloglycanes et produit cosmétique ou pharmaceutique contenant de tels extraits**
Verwendung von an Xyloglykanen angereicherten Tamarindensamenextrakten und solche Extrakte enthaltende kosmetische oder pharmazeutische Produkte
Use of tamarind seed extracts enriched in xyloglycans and cosmetics or pharmaceuticals containing them

(30) Priorité: 03.01.1995 FR 9500078
(43) Date de publication de la demande: 10.07.1996
(73) Titulaire: Cognis France S.A., 31360 Saint Martory (FR)
(72) Inventeur: Pauly, Gilles, F-54425 Pulnoy (FR)
(74) Mandataire: Nuss, Pierre

(56) Documents cités:
- DATABASE WPI Week 9517 Derwent Publications Ltd., London, GB; AN 95-128218 & JP-A-07 053 335 (KUREHA CHEM IND CO LTD) , 28 Février 1995
- DATABASE WPI Week 8432 Derwent Publications Ltd., London, GB; AN 84-198108 & JP-A-59 112 922 (ENDO A) , 29 Juin 1984
- DATABASE CHEMABS CHEMICAL ABSTRACTS SERVICE, COLUMBUS, OHIO, US AN: 115:203351, M. J. GIDLEY 'Structure and solution properties of tamarind seed polysaccharide'

## Description

La présente invention concerne l'utilisation d'extraits de graines de tamarin riches en xyloglycanes dans des applications cosmétiques et/ou pharmaceutiques.

Le tamarinier est un grand arbre à feuillage persistant appartenant à la famille des Légumineuses et dont les fruits sont de grosses gousses déhiscentes, à mésocarpe pulpeux, qui renferment chacune de 4 à 12 graines.

Il est déjà connu d'utiliser de diverses manières la pulpe des gousses et les graines de ces dernières.

Ainsi, la pulpe du fruit, riche en acides organiques libres et salifiés et renfermant un taux important de sucres simples, est utilisée dans l'alimentation (sauces, boissons) et en tant que laxatif doux.

La pulpe ou la chair des graines quant à elle contient principalement des polysaccharides, mais également des protéines, des lipides et des matières minérales.

Lesdits polysaccharides sont majoritairement des biopolymères xyloglycaniques ou xyloglycanes dont la composition en termes d'oses constitutifs est généralement la suivante :
- Xylose env. 18 %
- Galactose env. 23 %
- Glucose env. 55 %
- Arabinose env. 4 %

Il est déjà connu d'utiliser la poudre obtenue à partir des graines en tant qu'apprêt de la chaîne des fibres textiles, en tant qu'agent épaississant, en tant qu'agent de revêtement ou du surfaçage dans l'industrie du papier, du carton et dans l'industrie du textile.

De même, les polysaccharides extraits desdites graines de tamarin sont connus, en plus des applications précédentes, pour former des gels en présence de sucres, dans une plage étendue de pH.

Ainsi, lesdits polysaccharides sont mis en oeuvre en tant que substituants aux pectines de fruits dans la fabrication de confitures, de gelées et de marmelades et en tant que stabilisants dans les glaces et les mayonnaises.

Par ailleurs, l'utilisation de polysaccharides isolés spécifiques, tels que l'acide hyaluronique, la chitine, la cellulose, les carraghenates, les polysaccharides d'algues ou autres, est connue en cosmétique, du fait de leurs propriétés adoucissantes, hydratantes, protectrices et filmogènes.

WPI Week 9517 Derwent Publications Ltd., London, GB; AN 95-128218 & JP-A-07 053 335 (KUREHA CHEM IND CO LTD) , 28 Février 1995 divulgue l'utilisation des polysaccharides purs à usage topique.

Toutefois, les inventeurs de la présente invention ont constaté que, de manière inattendue et surprenante, les xyloglycanes extraits de graines de tamarin présentaient, du fait de leur composition particulière, d'une part, des propriétés immédiates plus variées et quantitativement nettement plus importantes que celles des polysaccharides précités connus, d'autre part, des effets à long termes supplémentaires et, enfin, une très bonne tolérance.

Ainsi, le principal objet de la présente invention concerne l'utilisation ou l'application d'extraits de graines de tamarin enrichis en xyloglycanes en tant qu'agent actif dans un produit ou une préparation cosmétique et/ou pharmaceutique à usage topique pour la peau et/ou les phanères.

Ces extraits de graines de tamarin riches en xyloglycanes pourront être obtenus par les techniques classiques d'extraction des polysaccharides, telle que l'extraction à chaud par des solutions aqueuses, hydroalcooliques ou alcooliques, en milieu acide ou neutre, sur des poudres délipidées ou non.

Il pourra, par exemple, procéder en réalisant les opérations suivantes : extraction de l'amande des graines de tamarin (après décorticage et élimination de l'enveloppe) et broyage de cette dernière, traitement de ladite poudre de graines avec un solvant adapté à l'élimination des lipides, extraction des xyloglycanes de la poudre de graines par dispersion de cette dernière dans une solution aqueuse acide, élimination de la fraction non solubilisée de la solution de xyloglycanes, récupération de la fraction de solution surnageante, riche en xyloglycanes, ajustement, le cas échéant, du pH et enfin, soumission éventuelle de ladite solution enrichie de xyloglycanes à au moins un traitement complémentaire en vue de modifier la présentation, la forme et/ou la constitution desdits xyloglycanes extraits, avant utilisation desdits xyloglycanes ou dudit extrait riche en xyloglycanes en tant qu'agent actif pour produit cosmétique et/ou pharmaceutique à usage topique (dermopharmaceutique) pour la peau et/ou les phanères.

Le solvant utilisé pour l'élimination des lipides de la poudre de graines est avantageusement de l'hexane (exemple non limitatif).

De plus, la solution aqueuse acide utilisée pour l'extraction consiste préférentiellement en une solution aqueuse d'un acide organique, tel que par exemple l'acide citrique, l'acide tartrique ou l'acide malique, diluée à entre 0,01 % et 10 %, préférentiellement à 0,2 %, l'extraction des xyloglycanes étant réalisée sous agitation vigoureuse et à chaud.

En vue de l'extraction des xyloglycanes, la poudre ou farine de graines de tamarin est avantageusement dispersée dans une quantité de solvant extractif 20 à 40 fois supérieur en poids à ladite poudre ou farine et la température de la solution au cours de l'extraction est maintenue à une valeur comprise entre 20° C et 140° C, de préférence entre 80° C et 100° C.

La durée de la phase d'extraction des xyloglycanes est, dans les conditions de température et d'agitation indiquées ci-dessus, variable entre 30 minutes et plusieurs heures. Toutefois, l'essentiel de l'extraction est généralement achevé au bout de 40 minutes.

Après cette phase d'extraction, la fraction non solubilisée de la poudre ou de la farine est séparée de l'extrait liquide contenant les xyloglycanes, par centrifugation, tamisation ou analogue.

En outre, la fraction de solution riche en xyloglycanes peut, après prélèvement sélectif de l'extrait liquide contenant les xyloglycanes, être ajustée à une valeur de pH adaptée à son utilisation ultérieure.

Le traitement complémentaire peut comprendre la déshydratation de la solution enrichie en xyloglycanes par lyophilisation ou atomisation ou l'extraction directe et la purification sélective des xyloglycanes à partir de ladite solution enrichie, par précipitation par un solvant organique suivie d'une élimination du solvant et d'un séchage.

Le solvant organique utilisé pour la précipitation peut avantageusement consister en de l'alcool éthylique mélangé à la solution enrichie en xyloglycanes avec un rapport volumique de 1. Le précipité fibreux, issu de ladite précipitation, est ensuite récupéré par exemple par filtration ou tamisage et soumis à un pressage pour en éliminer au maximum le solvant d'imbibition, avant de procéder à son séchage.

L'incorporation des extraits de graines de tamarin riches en xyloglycanes dans des produits ou des préparations cosmétiques et/ou pharmaceutiques à usage cutané, capillaire ou analogue peut être réalisée sous forme de solution ou de suspension aqueuse, de suspension hydropolyolique ou encore à l'état déshydraté, après remise en solution effectuée de manière extemporanée, l'incorporation s'effectuant préférentiellement après redissolution dans un solvant adéquat ou intégration dans une formulation autorisant l'expression des propriétés spécifiques des xyloglycanes.

Toutefois, en plus des traitements complémentaires prévus ci-dessus ou en variante par rapport à ces derniers, il peut également être prévu d'autres traitements de la solution riche en xyloglycanes ou des extraits enrichis en xyloglycanes sous forme solide, comprenant des opérations de modification de la forme et/ou de la constitution des xyloglycanes extraits telles que, par exemple, l'hydrolyse partielle ou totale, la polymérisation, la réticulation par des aldéhydes, la condensation et le greffage avec des acides gras, la méthylation ou encore la vectorisation, par exemple par encapsulation sous forme de microsphères ou de microcapsules, de liposomes, de nano-capsules, de nano-sphères, de micro-éponges, de clathrates ou autres.

Conformément à l'invention, les extraits de graines de tamarin enrichis en xyloglycanes sont présents dans le produit cosmétique et/ou pharmaceutique à usage topique selon une fraction totale en poids comprise entre 0,05 % et 100 %, préférentiellement une fraction totale en poids comprise entre 1 % et 25 %.

Ces extraits enrichis en xyloglycanes comportent généralement entre 3 % et 10 % de xyloglycanes purs, préférentiellement environ 5 %. Toutefois, ces extraits pourront également présenter notamment une concentration en xyloglycanes nettement plus élevée et, dans le cas extrême, être constitués de xyloglycanes pratiquement purs.

La présente invention a également pour objet une préparation ou un produit cosmétique et/ou pharmaceutique à usage topique pour la peau et/ou les phanères, comportant entre 0,05 % et 100 % en poids d'extraits de graines de tamarin enrichis en xyloglycanes.

Préférentiellement, la préparation ou le produit précités pourra consister en une composition traitante comportant entre 1 % et 25 % en poids d'extrait de graines de tamarin enrichis en xyloglycanes, ces derniers présentant une composition fractionnaire en oses constitutifs identique à celle existant dans l'amande des graines de tamarin à l'état naturel.

L'objet de la présente invention pourra être mis en oeuvre plus généralement dans tout support cosmétique et/ou dermo-pharmaceutique acceptable pour une application sur la peau ou les phanères, et compatible avec l'activité des xyloglycanes.

Lors de la mise en oeuvre d'un produit cosmétique et/ou pharmaceutique à usage topique conforme à l'invention et destiné à une application cutanée, il a été constaté immédiatement une sensation de douceur et de velouté, un confort cutané amélioré ainsi que l'apparition d'un aspect satiné, notamment lors de l'application sur une peau sensible ou sèche.

Ces effets résultent directement des propriétés filmogènes, adoucissantes, lissantes, hydratantes et réparatrices, à action immédiate des xylogycanes extraits des graines de tamarin.

On aboutit ainsi à une amélioration immédiate des qualités tactiles de la peau telles que mentionnées ci-dessus, grâce à la formation en surface, d'un réseau macromoléculaire fortement hydraté, à caractère hydratant, élastifiant et viscoélastique.

En outre, on observe également un effet protecteur et conditionneur, voire substantif, sans action occlusive.

Par ailleurs, en plus des effets immédiats précités, les produits cosmétiques à application cutanée selon l'invention présentent également des actions à long terme de nature immunomodulatrice et immunostimulante.

Par conséquent, les xyloglycanes extraits des graines de tamarin trouveront une application privilégiée, d'une part, dans le cadre des produits de soins de la peau tels que des produits anti-vieillissement, des produits après-rasage, des produits solaires ou après-solaires et, d'autre part, dans le cadre des produits de maquillage traitant tels que les crèmes teintées, les fonds de teint, les rouges à lèvres ou analogues.

Toutefois, selon une variante d'application, la présente invention concerne également les produits cosmétiques pour le domaine du traitement capillaire, tels que les shampooings, les baumes ou les lotions, et l'intégration des xyloglycanes extraits de graines de tamarin dans ces produits permet de rendre les cheveux plus doux, plus lisses et faciles à coiffer, du fait notamment de l'effet filmogène obtenu.

A titre d'exemples non limitatifs de réalisations pratiques de l'invention, on décrira ci-après différent(e)s produits ou préparations cosmétiques ou pharmaceutiques à usage topique pour la peau ou les phanères, comprenant des extraits de graines de tamarin, enrichis en xyloglycanes.

### Exemple 1:

Un produit cosmétique sous forme de crème pour le visage conforme à l'invention pourra, par exemple, présenter une composition pondérale, constituée à partir des fractions A, B et C suivantes, telle qu'indiquée ci-après.

### Fraction A :

- Alcool cétostéarylique (et) ceteareth-20 5,00 %
- Stéarate de glycérol (et) stéarate de PEG-100 3,50 %
- Stéarate de glycérol 2,50 %
- Alcool cétostéarylique 2,00 %
- Huile de paraffine 3,00 %
- Octyldodécanol 5,00 %

### Fraction B :

- Eau qsp 100,00 %
- Conservateur qs

### Fraction C :

- Extrait de graines de tamarin enrichi en xyloglycanes 5,00 %

Le procédé de préparation et de fabrication de la crème pour le visage ci-dessus consiste essentiellement à chauffer la fraction A et la fraction B à 80° C, à introduire la fraction A dans la fraction B sous agitation (au moyen d'une turbine) en maintenant la température précitée, à refroidir le mélange à 45° C en maintenant l'agitation, à y ajouter la fraction C et enfin à ramener la préparation finale obtenue à température ambiante, sous agitation planétaire.

### Exemple 2 :

Un produit cosmétique sous forme de gel pour le visage conforme à l'invention pourra, par exemple, présenter une composition pondérale, constituée par les fractions A, B, C, D et E, telle qu'indiquée ci-après.

### Fraction A :

- Eau qsp 100,00 %
- Conservateur qs
- Glycérine 3,00 %
- Copolyol de Dimethicone 2,00 %

### Fraction B :

- Gomme xanthane 0,40 %

### Fraction C :

- Extrait de graines de tamarin enrichi en xyloglycanes 10,00 %

### Fraction D :

Polyacrylamide (et) C13-14 isoparaffine (et) laureth-7 4,00 %

### Fraction E :

Parfum 0,40 %

Le procédé de préparation et de fabrication du gel pour le visage susvisé consiste essentiellement à préparer la fraction A à 50° C, à disperser la fraction B dans la fraction A sous agitation (au moyen d'une turbine), à laisser refroidir le mélange A + B à température ambiante, à y ajouter successivement la fraction C et la fraction D en réalisant simultanément une homogénéisation jusqu'à obtention d'un gel homogène, à ajouter la fraction E et enfin à homogénéiser et à désaérer la préparation finale obtenue.

### Exemple 3 :

Un produit cosmétique sous forme de lait pour le corps conforme à l'invention pourra, par exemple, présenter une composition pondérale, constituée à partir des fractions A, B, C, D, E et F suivantes, telle qu'indiquée ci-après.

### Fraction A :

- Alcool cétostéarylique (et) ceteareth-20 1,500 %
- Stéarate de glycérol (et) stéarate de PEG-100 1,000 %
- Stéarate de propylène glycol autoémulsionnable 2,000 %
- Huile de paraffine 4,000 %
- Myristate d'isopropyle 2,000 %
- Triglycéride caprylique/caprique 6,000 %

### Fraction B :

- Eau qsp 100,000 %
- Propylène glycol 2,000 %
- Conservateur qs %

### Fraction C :

- Extrait de graines de tamarin enrichi en xyloglycanes 5,000 %

### Fraction D :

- Gomme xanthane 0,400 %
- Conservateur 0,060 %
- Eau 19,540 %

### Fraction E :

- Silicate de magnésium et d'aluminium 0,900 %
- Conservateur 0,054 %
- Eau 17,049 %

### Fraction F :

- Parfum 0,400 %

Le procédé de préparation et de fabrication du lait pour le corps mentionné ci-dessus consiste essentiellement à préparer séparément les fractions D et E, à préparer la fraction B à 80° C, à y ajouter les fractions D et E refroidies à température ambiante et à maintenir le mélange B + D + E à 80° C. Ensuite, il y a lieu de préparer la fraction A 80° C, d'ajouter sous agitation (au moyen d'une turbine) ladite fraction A dans le mélange B + D + E à 80° C, d'opérer un refroidissement dudit mélange A + B + D + E, d'ajouter la fraction C à ce dernier lorsqu'il est refroidi à environ 45° C à 50° C, ce sous agitation (au moyen d'une turbine). Enfin, il y a lieu d'ajouter la fraction F lorsque le mélange A + B+ C + D + E précité atteint 40° C, puis de refroidir la préparation finale ainsi obtenue jusqu'à température ambiante, ce sous agitation planétaire.

### Exemple 4

Un produit cosmétique sous forme de shampooing traitant conforme à l'invention pourra, par exemple, présenter une composition pondérale, constituée à partir des fractions A, B, C, D et E suivantes, telle qu'indiquée ci-après.

### Fraction A :

- Eau qsp 100,00 %
- Conservateur qs
- Chlorure d'hydroxypropyltrimonium / hydroxypropyle guar gomme 0,40 %

### Fraction B :

- Sulfate laureth de sodium (et) lauryl polyglucose 25,00 %

### Fraction C :

- Cocamidopropyle bétaïne 8,00 %
- Cocoate de glyceryl PEG 7 0,50 %
- Diméthicone copolyol 1,00 %
- Parfum 0,30 %

### Fraction D :

- Solution aqueuse de citrate de sodium qsp pH =6

### Fraction E :

- Extrait de graines de tamarin enrichi en xyloglycanes 5,00 %

Le procédé de préparation et de fabrication du shampooing traitant dont la composition est indiquée ci-dessus consiste essentiellement à préparer la fraction A à 80° C sous agitation (au moyen d'une turbine), à refroidir ladite fraction A à température ambiante, à y ajouter successivement les fractions B, C, D et E, à température ambiante et enfin à filtrer le mélange final obtenu avec un filtre de 80 µm.

### Exemple 5

Un produit cosmétique sous forme d'émulsion capillaire conforme à l'invention pourra, par exemple, présenter une composition pondérale, constituée à partir des fractions A, B, C, D et E suivantes, telle qu'indiquée ci-dessous.

### Fraction A :

- Stéarate de glycérol (et) stéarate de PEG-100 2,50 %
- Alcool oléylique 2,00 %
- Diméthicone 1,00 %
- Cyclométhicone 2,00 %

### Fraction B :

- Eau qsp 100,00 %
- Conservateur qs
- Chlorure d'hydroxypropyltrimonium guar gomme 1,00 %
- Gomme guar 1,00 %

### Fraction C :

Extrait de graines de tamarin enrichi en xyloglycanes 5,00 %

### Fraction D :

Solution acide citrique / citrate de sodium qs pH = 6

Le procédé de préparation et de fabrication de l'émulsion capillaire décrite ci-dessus consiste essentiellement à préparer la fraction A à 75° C, à préparer séparément la fraction B sous agitation (au moyen d'une turbine) à 75° C, jusqu'à obtention d'une constitution homogène et d'une dispersion parfaite des gommes, à ajouter la fraction A dans la fraction B, en maintenant l'agitation et à refroidir le mélange A + B jusqu'à 50° C, en conservant toujours l'agitation. Ensuite, il y a lieu d'ajouter la fraction C au mélange A + B précité à 50° C, d'homogénéiser le mélanger A + B + C résultant, de refroidir ce dernier à température ambiante sous agitation planétaire et enfin d'ajuster le pH du mélange précité avec la fraction D.

### Exemple 6

Un produit pharmaceutique à usage topique sous forme de pommade, conforme à l'invention, pourra, par exemple, présenter une composition pondérale telle qu'indiquée ci-dessous.
- Oxyde de zinc 5 %
- Eau 10 %
- Lanoline 35 %
- Vaseline 35 %
- Extrait de graines de tamarin enrichi en xyloglycanes 15 %

Le procédé de préparation et de fabrication de la pommade, dont la composition est indiquée ci-dessus, consiste essentiellement à faire fondre le mélange de lanoline et de vaseline à une température d'environ 60° C, à incorporer l'oxyde de zinc finement retamisé et enfin à ajouter la solution constituée par le mélange de l'eau avec l'extrait de graines de tamarin enrichi en xyloglycanes.

Bien entendu, l'invention n'est pas limitée aux modes de réalisation décrits. Des modifications restent possibles, notamment du point de vue de la constitution des divers éléments ou par substitution d'équivalents techniques, sans sortir pour autant du domaine de protection de l'invention.

## Revendications

1. Utilisation d'extraits de graines de tamarin enrichis en xyloglycanes en tant qu'agent actif pour la fabrication d'un produit pharmaceutique à usage topique pour la peau et/ou les phanères.

2. Utilisation, selon la revendication 1, **caractérisée en ce que** les extraits de graines de tamarin enrichis en xyloglycanes sont présents dans le produit cosmétique et/ou pharmaceutique à usage topique selon une fraction totale en poids comprise entre 0,05 % et 100 %, ces extraits comportant entre 3 et 10% en poids de xyloglycanes purs.

3. Utilisation, selon la revendication 2, **caractérisée en ce que** les extraits de graines de tamarin enrichis en xyloglycanes sont présents dans le produit cosmétique et/ou pharmaceutique à usage topique avec une fraction totale en poids comprise entre 1 % et 25 %.

4. Utilisation non-therapeutique d'extraits de graines de tamarin enrichis en xyloglycanes en tant qu'agent actif dans un produit cosmétique à usage topique pour la peau et/ou les phanères ces extraits comportant entre 3 et 10% en poids de xyloglycanes purs.

5. Utilisation, selon la revendication 1 à 3 pour l'immunostimulation et/ou l'immunomodulation.

6. Utilisation, selon l'une quelconque des revendications 4, **caractérisée en ce que** les extraits de graines de tamarin enrichis en xyloglycanes sont présents dans le produit cosmétique à usage topique selon une fraction totale en poids comprise entre 0,05 % et 100 %, ces extraits comportant entre 3 et 10% en poids de xyloglycanes purs.

7. Utilisation, selon la revendication 6, **caractérisée en ce que** les extraits de graines de tamarin enrichis en xyloglycanes sont présents dans le produit cosmétique à usage topique avec une fraction totale en poids comprise entre 1 % et 25 %.

8. Produit cosmétique ou pharmaceutique pour la peau et/ou les phanères, **caractérisé en ce qu'**il comporte entre 0,05 % et 100 % en poids d'extrait de graines de tamarin enrichis en xyloglycanes, ces extraits comportant entre 3 et 10% en poids de xyloglycanes purs.

9. Produit selon la revendication 8, **caractérisé en ce qu'**il consiste en une composition traitante comportant entre 1 % et 25 % en poids d'extrait de graines de tamarin enrichis en xyloglycanes, ces derniers présentant une composition fractionnaire en oses constitutifs identique à celle existant dans l'amande des graines de tamarin à l'état naturel.

## Claims

1. Use of extracts of tamarind seeds enriched in zyloglycans as active ingredient for the manufacture of a pharmaceutical product for typical use for the skin and/or superficial body growths.

2. Use according to claim 1, **characterised in that** the extracts of tamarind seeds enriched in zyloglycans are present in the cosmetic and/or pharmaceutical product for topical use in a total content of between 0.05% and 100% by weight, these extracts comprising between 3 and 10% by weight of pure zyloglycans.

3. Use according to claim 2, **characterised in that** the extracts of tamarind seeds enriched in zyloglycans are present in the cosmetic and/or pharmaceutical product for topical use in a total content of between 1% and 25% by weight.

4. Non-therapeutic use of extracts of tamarind seeds enriched in zyloglycans as active ingredient in a cosmetic product for topical use for the skin and/or superficial body growths, these extracts comprising between 3 and 10% by weight of pure zyloglycans.

5. Use according to claims 1 to 3, for immunostimulation and/or immunomodulation.

6. Use according to claim 4, **characterised in that** the extracts of tamarind seeds enriched in zyloglycans are present in the cosmetic product for topical use in a total content of between 0.05% and 100%: these extracts comprising between 3 and 10% by weight of pure zyloglycans.

7. Use according to claim 6, **characterised in that** the extracts of tamarind seeds enriched in zyloglycans are present in the cosmetic product for topical use in a total content of between 1% and 25% by weight.

8. Cosmetic or pharmaceutical product for the skin and/or superficial body growths, **characterised in that** it comprises between 0.05% and 100% by weight of extract of tamarind seeds enriched in zyloglycans: these extracts comprising between 3 and 10% by weight of pure zyloglycans.

9. Product according to claim 8, **characterised in that** it consists of a treating composition comprising between 1% and 25% by weight of extract of tamarind seeds enriched in zyloglycans, the latter having a fractional composition of constituent monosaccharides which is identical to that in the kernel of the tamarind seeds in the natural state.

## Patentansprüche

1. Verwendung von mit Xyloglykanen angereicherten Tamarindensamenextrakten als Wirkstoff zur Herstellung eines pharmazeutischen Produkts zur örtlichen Verwendung für die Haut und/oder die Oberhautanhangsgebilde.

2. Verwendung nach Anspruch 1, **dadurch gekennzeichnet, dass** die mit Xyloglykanen angereicherten Tamarindensamenextrakte in dem kosmetischen und/oder pharmazeutischen Produkt zur örtlichen Verwendung gemäß einem Gesamtgewichtsanteil zwischen 0,05% und 100% vorliegen, wobei diese Extrakte zwischen 3 und 10 Gewichtsprozent reine Xyloglykane enthalten.

3. Verwendung nach Anspruch 2, **dadurch gekennzeichnet, dass** die mit Xyloglykanen angereicherten Tamarindensamenextrakte in dem kosmetischen und/oder pharmazeutischen Produkt zur örtlichen Verwendung mit einem Gesamtgewichtsanteil zwischen 1% und 25% vorliegen.

4. Nicht-therapeutische Verwendung von mit Xyloglykanen angereicherten Tamarindensamenextrakten als Wirkstoff in einem kosmetischen Produkt zur örtlichen Verwendung für die Haut und/oder die Exoskeleta, wobei diese Extrakte zwischen 3 und 10 Gewichtsprozent reine Xyloglykane enthalten.

5. Verwendung nach Anspruch 1 bis 3 zur Immunstimulation und/oder Immunmodulation.

6. Verwendung nach Anspruch 4, **dadurch gekennzeichnet, dass** die mit Xyloglykanen angereicherten Tamarindensamenextrakte in dem kosmetischen Produkt zur örtlichen Verwendung gemäß einem Gesamtgewichtsanteil zwischen 0,05% und 100% vorliegen, wobei diese Extrakte zwischen 3 und 10 Gewichtsprozent reine Xyloglykane enthalten.

7. Verwendung nach Anspruch 6, **dadurch gekennzeichnet, dass** die mit Xyloglykanen angereicherten Tamarindensamenextrakte in dem kosmetischen Produkt zur örtlichen Verwendung mit einem Gesamtgewichtsanteil zwischen 1% und 25% vorliegen.

8. Kosmetisches oder pharmazeutisches Produkt für die Haut und/oder die Exoskeleta, **dadurch gekennzeichnet, dass** es zwischen 0,05 und 100 Gewichtsprozent mit Xyloglykanen angereichertes Tamarindensamenextrakt enthält, wobei diese Extrakte zwischen 3 und 10 Gewichtsprozent reine Xyloglykane enthalten.

9. Produkt nach Anspruch 8, **dadurch gekennzeichnet, dass** es aus einer Behandlungszusammensetzung besteht, die zwischen 1 und 25 Gewichtsprozent Tamarindensamenextrakt enthält, der mit Xyloglykanen angereichert ist, wobei diese letzteren eine Fraktionszusammensetzung an Bestandteilsmonosacchariden aufweisen, die zu jener identisch ist, die im Kern der Tamarindensamen im natürlichen Zustand existiert.
